# EUROPÄISCHE PATENTSCHRIFT

(11) **EP 0 721 928 B1**
(45) Veröffentlichungstag und Bekanntmachung des Hinweises auf die Patenterteilung: **22.12.1999**
(21) Anmeldenummer: 95120535.0
(22) Anmeldetag: 27.12.1995
(51) Int. Cl.: C07C 29/149, C07C 67/465, C07C 31/20

(54) **Verfahren zur Herstellung von aliphatischen alpha, omega-Diolen**
Method for the preparation of aliphatic alpha, omega-diols
Procédé pour la préparation des alpha, oméga-dioles

(30) Priorität: 13.01.1995 DE 19500783
(43) Veröffentlichungstag der Anmeldung: 17.07.1996
(73) Patentinhaber: BAYER AG, 51368 Leverkusen (DE)
(72) Erfinder: Darsow, Gerhard, Dr., D-47804 Krefeld (DE); Petruck, Gerd-Michael, Dr., D-40699 Erkrath (DE); Alpers, Heinz-Jürgen, D-47829 Krefeld (DE)

(56) Entgegenhaltungen:
- WO-A-82/03854
- DE-B- 1 023 750

## Beschreibung

Die vorliegende Erfindung betrifft ein Verfahren zur Herstellung von aliphatischen α,ω-Diolen mit 4 bis 12 C-Atomen aus aliphatischen α,ω-Dicarbonsäuren mit 4 bis 12 C-Atomen, bei welchem nur sehr geringe Mengen der üblicherweise bei der Hydrierung solcher Säuren oder ihrer Esterderivate als Nebenprodukte entstehenden Monoalkohole der C-Zahlen 1 bis 12 sowie keine C₄-C₁₂-Lactone und keine C₄-C₁₂-ω-Hydroxycarbonsäuren gebildet werden. Hierzu wird auf den Einsatz von Monoalkoholen für die Esterherstellung verzichtet, indem man zunächst aus der einzusetzenden Carbonsäure und dem herzustellenden Diol ein oligomeres Diol-Dicarboxylat herstellt, das anschließend ohne weitere destillative Reinigung katalytisch in der Flüssigphase mit Wasserstoff hydriert wird. Die genannten Umstände machen das erfindungsgemäße Verfahren ökologisch und technisch vorteilhaft.

Aliphatische α,ω-Diole, beispielsweise Hexandiol-1,6, sind wichtige Monomere für die Produktion von thermoplastischen Polyestern sowie von Polyurethanen mit besonderen mechanischen und chemischen Eigenschaften. Hierzu können solche Diole in reiner Form oder als Gemische mehrerer Diole mit unterschiedlichen Kettenlängen im C₄-C₁₂-Bereich eingesetzt werden.

Zur Herstellung beispielsweise von Hexandiol-1,6 ist es bekannt, Adipinsäure oder ihre Salze direkt, in wäßriger Lösung oder in einem organischen Lösungsmittel diskontinuierlich (DE-OS 26 05 107; GB 1.300.889) oder kontinuierlich (DE-OS 23 21 101) zu hydrieren, wobei neben Hexandiol-1,6 stets größere Anteile an Caprolacton, ω-Hydroxy-capronsäure sowie Monoalkohole der C-Zahlen 1 bis 6 gebildet werden. Zum gleichen Zweck ist es weiterhin bekannt, Adipinsäure mit Monoalkoholen zu einem Di-n-alkyladipinat zu verestern und dieses in der Gasphase zu Hexandiol-1,6 zu hydrieren (WO 82/03854). Es ist außerdem bekannt, Adipinsäure diskontinuierlich mit Diolen, wie Hexandiol-1,6, zu einem Gemisch höherer Ester zu verestern und dieses in einem diskontinuierlichen Autoklavenprozeß zu Hexandiol-1,6 und ω-Hydroxy-capronsäureester zu hydrieren, wobei als Hydrierkatalysator ein pulverförmiger Kupferchromit-Katalysator zum Einsatz gelangt (DE-AS 10 23 750).

Die Erfindung betrifft ein Verfahren zur Herstellung von aliphatischen α,ω-Diolen mit 4 bis 12 C-Atomen aus aliphatischen α,ω-Dicarbonsäuren mit 4 bis 12 C-Atomen durch Oligoveresterung der genannten Dicarbonsäuren mit den genannten Diolen und katalytische Hydrierung des entstandenen Oligoesters in der Flüssigphase, das dadurch gekennzeichnet ist, daß der Oligoester kontinuierlich bei 180 bis 250°C und einem H₂-Druck von 100 bis 400 bar wobei die H₂-Menge das 20- bis 100-fache der stöchiometrisch nötigen Menge beträgt, an einem stückigen reduzierten Katalysator, vor der Reduktion bestehend aus verpreßten Pulvern von Cu-, Zn- und Al-Oxiden mit einem oder ohne einen Gehalt von mindestens einem Oxid von Metallen der Eisengruppe des Periodensystems der Elemente (Mendelejew) oder des Mangans, hydriert wird.

Der Reaktionsverlauf läßt sich anhand der Bildung von Hexandiol-1,6 aus Adipinsäure unter Benutzung von Hexandiol-1,6 zur Oligoveresterung durch das folgende Reaktionsschema veranschaulichen:

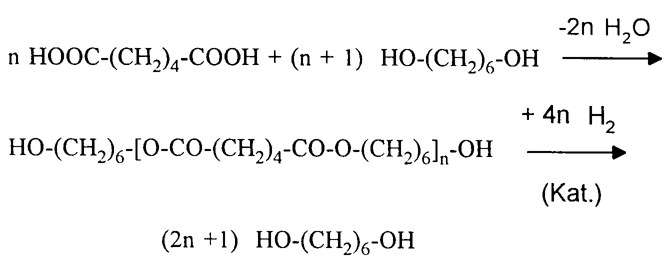

In den bisher bekanntgewordenen Verfahren treten stets größere Mengen an Nebenprodukten auf, die einen erheblichen Aufwand bei der Reindarstellung des Reaktionsproduktes verursachen. Eine Recyclisierung ist nicht immer möglich, da die Hydrierung zum gewünschten Endprodukt, wie oben bereits erwähnt, nicht immer glatt verläuft und daher zu einem höheren Niveau an im Kreis geführten Nebenprodukten führen würde. Eine grundsätzliche Schwierigkeit bereitet ferner der zur Veresterung bislang eingesetzte Monoalkohol, der grundsätzlich vom Endprodukt abgetrennt werden muß. Das Festhalten an Monoalkoholen zur Veresterung erklärt sich ganz offenbar aus der Tatsache, daß bei der Fortentwicklung des Hydrierverfahrens von der flüssigen zur gasförmigen Phase ein leicht verdampfbarer Ester zur Verfügung stehen mußte. Für solche leicht verdampfbaren Ester aus Monoalkoholen spricht ferner die Tatsache, daß sie in übersichtlicher Weise rein dargestellt werden können; dies schien sehr wichtig zu sein, da bei Hydrierungen verunreinigte Ausgangsmaterialien in der Regel zu Schwierigkeiten führen. Solche Ester aus Dicarbonsäuren und niederen Alkoholen, beispielsweise Methanol, Ethanol oder Propanol, erfordern jedoch in der Regel die Benutzung eines Veresterungskatalysators und eines Wasserschleppmittels, wie beispielsweise Toluol, die beide weitere systemfremde Stoffe darstellen, die abgetrennt werden müssen. Die Benutzung eines Wasserschleppmittels, das als azeotropes Gemisch mit dem gebildeten Reaktionswasser aus dem Reaktionsgemisch abdestilliert, vom Wasser abgetrennt und wieder in das Reaktionsgemisch zurückgeführt werden muß, bedeutet einen zusätzlichen apparativen Aufwand. Weiterhin ist verhältnismäßig viel Energie aufzuwenden, um das Schleppmittel, dessen Menge ein Vielfaches der gebildeten Wassermenge beträgt, wiederholt aus dem Reaktionsgemisch abzudestillieren. Schließlich muß der Dicarbonsäure-Dialkylester vor der katalytischen Hydrierung durch Destillation oder andere umständliche Reinigungsmaßnahmen sorgfältig vom Veresterungskatalysator befreit werden. Nach der Hydrierung muß der Monoalkohol mit technischem Aufwand von den Reaktionsprodukten getrennt, auf reinen Alkohol aufgearbeitet und wieder dem Veresterungsprozeß zugeführt werden, wobei nicht zu vermeidende Alkoholverluste ergänzt werden müssen.

Wollte man eine Dicarbonsäure mit höhersiedenden Alkoholen, beispielsweise solchen mit C-Zahlen von größer als 3, verestern, um sie als Dialkylester in der Gasphase zu hydrieren, kann man möglicherweise auf den Veresterungskatalysator und das Schleppmittel verzichten, benötigt aber weiterhin als reaktionsfremden Stoff den relativ teuren, höhersiedenden Monoalkohol, der ebenfalls wieder auf reinen Alkohol aufgearbeitet und wieder dem Veresterungsprozeß zugeführt werden muß, wobei auch hier nicht zu vermeidende Alkoholverluste ergänzt werden müssen, die wegen des höheren Preises solcher Alkohole die Wirtschaftlichkeit eines solchen Verfahrens empfindlich treffen. Wegen der höheren Siedepunkte ist zusätzlich jeder Destillationsaufwand größer.

Anders und vorteilhaft liegt der Fall, wenn zur Veresterung der Dicarbonsäure mit 4 bis 12 C-Atomen ein Diol mit 4 bis 12 C-Atomen eingesetzt wird, da in einem solchen Falle weder Veresterungskatalysatoren noch Schleppmittel erforderlich sind und da das Veresterungsdiol als systemeigener Stoff im Reaktionsprodukt verbleiben kann. Für die obengenannten Verwendungszwecke ist es in vielen Fällen zulässig, ein Gemisch von Diolen mit verschiedener Kettenlänge einzusetzen; dadurch ist es nicht erforderlich, zur Oligoveresterung der Dicarbonsäure ein Diol mit der gleichen Anzahl von C-Atomen einzusetzen. Vielmehr ist es erfindungsgemäß zulässig, beispielsweise Adipinsäure außer mit Hexandiol-1,6 auch mit Butandiol-1,4, Octandiol-1,8 und anderen Diolen umzusetzen und dann zu einem Gemisch von beispielsweise C₆-Diol mit C₄-Diol oder mit C₈-Diol zu erhalten. Um jedoch α,ω-Diole als Reaktionsprodukte mit einheitlicher C-Zahl zu erhalten, wird die umzusetzende Dicarbonsäure mit einem Diol gleicher C-Zahl oligoverestert und hydriert, beispielsweise Adipinsäure mit Hexandiol-1,6, Bernsteinsäure mit Butandiol-1,4, Suberinsäure (Korksäure) mit Octandiol-1,8 usw.

Erfindungsgemäß einsetzbare Dicarbonsäuren mit geradzahligen oder ungeradzahligen C-Atomen, wobei die C-Atome der beiden Carboxylgruppen stets mitgezählt werden, sind beispielsweise: Bernsteinsäure, Glutarsäure, Adipinsäure, Pimelinsäure, Suberinsäure (Korksäure), Heptandicarbonsäure, Octandicarbonsäure und Decandicarbonsäure. Sie sind bekannt und aus natürlichen oder synthetischen Quellen zugänglich.

Erfindungsgemäß herzustellende und im Schritt der Oligoveresterung einsetzbare α,ω-Diole mit geradzahligen oder ungeradzahligen C-Atomen sind beispielsweise Butandiol-1,4, Pentandiol-1,5, Hexandiol-1,6, Heptandiol-1,7, Octandiol-1,8, Decandiol-1,10 und Dodecandiol-1,12.

Das erfindungsgemäße Verfahren ist besonders wichtig für die Herstellung von Hexandiol-1,6 durch Oligoveresterung von Adipinsäure mit Hexandiol-1,6 und anschließende Hydrierung.

Zur Herstellung von Oligoestern, wie sie im erfindungsgemäßen Verfahren benötigt werden, beispielsweise zur Herstellung von Hexandiol-1,6-adipinat, wird normalerweise ein diskontinuierliches Verfahren (Batch-Prozeß) angewendet, bei dem die Dicarbonsäure und das Diol in einem Reaktor mit oder ohne Katalysator verestert und das bei der Veresterung entstehende Wasser mit oder ohne Schleppmittel aus dem Reaktionsgemisch entfernt wird. Solche Oligoester sind erfindungsgemäß einsetzbar. Diskontinuierliche Verfahren der geschilderten Art haben jedoch den Nachteil, daß ihre Kapazität, relativ zum Reaktionsvolumen, klein ist und somit ein Bedarf nach großen Reaktoren und Lagertanks besteht Der Energieverbrauch ist unökonomisch, der Personalbedarf verhältnismäßig hoch.

Kontinuierliche Veresterungsverfahren mit mehreren in Kaskade geschalteten Veresterungsreaktoren vermeiden einen Teil dieser Nachteile. Es ist daher eine bevorzugte Variante des erfindungsgemäßen Verfahrens, daß die Oligoveresterung neben der diskontinuierlichen Weise auch in quasi-kontinuierlicher Weise und damit außer in nur einer Veresterungsstufe auch in bis zu vier Veresterungsstufen, bevorzugt quasi-kontinuierlich in zwei oder drei Veresterungsstufen unter destillativer Entfernung des Reaktionswassers durchgeführt wird. Als Temperaturbereich kommt hierbei der Bereich von 100 bis 240°C und als Druckbereich der von 1500 bis 100 mbar in Frage. Auch diese Durchführung in mehr als einer, beispielsweise 2 bis 4 Veresterungsstufen, bevorzugt 2 oder 3 Veresterungsstufen, vermag alleine jedoch kaum, die relativ langen Reaktionszeiten von beispielsweise 12 Stunden und mehr wesentlich zu verkürzen, wenn nicht durch technische Maßnahmen, beispielsweise durch schnellaufende Rührsysteme, die Wasseraustragung aus dem Reaktionsgemisch beschleunigt wird. Eine weitere Beschleunigung der Wasseraustragung ist beispielsweise die durch Einblasen eines Treibgases, beispielsweise durch Einblasen von Stickstoff.

Wesentlich wirksamer ist es dagegen und stellt somit eine weitere bevorzugte Variante des erfindungsgemäßen Verfahrens dar, eine Reaktorkaskade mit einer Temperaturkaskade zu kombinieren. So hat es sich als nützlich erwiesen, die Veresterungsreaktion der schmelzflüssigen Ausgangskomponenten zunächst bei einer relativ niedrigen Reaktionstemperatur von beispielsweise 110 bis 150°C zu starten um sie in einer zweiten und weiteren Veresterungsstufe bei erhöhter Temperatur fortzusetzen, wobei die Temperatur von Stufe zu Stufe um 10 bis 40°C, bevorzugt um 15 bis 30°C gesteigert wird. Innerhalb jeder Stufe kann die Temperatur nicht nur geringfügig schwanken, sondern gezielt um 5 bis 30°C gesteigert werden. Zusätzlich zur Temperaturkaskade kann noch eine Veränderung des Druckes treten, wobei grundsätzlich im allgemeinen Bereich von 1500 bis 100 mbar eine Druckabsenkung mit steigender Temperatur einhergeht. So kann beispielsweise bei mehrstufiger Oligoveresterung die erste Stufe bei Normaldruck, die letzte Stufe bei 200 bis 600 mbar und im Falle von 3 oder 4 Stufen die mittlere(n) Stufe(n) bei 400 bis 100 mbar durchgeführt werden.

Bei einstufiger Fahrweise und ebenso bei Verwendung mehrerer Reaktoren kann die Oligoveresterung so geführt werden, daß in einem gewissen Zeittakt die Reaktoren zeitlich versetzt mit Veresterungsgemisch gefüllt werden. So steht jeweils nach kürzerer Zeit, als für die gesamte Veresterung benötigt wird, eine neue Charge Oligoester für die folgende Hydrierung zur Verfügung. Bei mehrstufiger Veresterung in der beschriebenen Art wird das Veresterungsgemisch zur Durchführung der folgenden Veresterungsstufe von einem Reaktor in den anderen übergeführt. Der jeweils freigewordene Reaktor kann sodann mit einer Folgecharge gefüllt werden. Auch hierbei steht nach einer kürzeren Zeit, als sie für die gesamte Oligoveresterung erforderlich ist, eine neue Charge Oligoester für die nachfolgende Hydrierung zur Verfügung. Auf diese Weise ist eine quasikontinuierliche Oligoveresterung möglich.

Die für die Oligoveresterung in Frage kommenden Reaktionsapparate bestehen im allgemeinen aus einem oder mehreren Reaktoren aus säurefestem Material, die mit einem wirksamen Rührer und einer aufgesetzten Destillationskolonne üblicher Bauart mit 8 bis 15 Böden ausgerüstet ist.

Wollte man beispielsweise eine Dicarbonsäure unter den angegebenen Veresterungsbedingungen mit einem Diol im molaren Verhältnis von 1:1 verestern, so erhielt man ein Gemisch von Oligoestern unterschiedlicher Oligomerisierungsgrade (= Zahl der Moleküle Dicarbonsäure im Oligoester), deren Molmassenverteilung recht genau einer Glockenkurve folgt. Der mittlere Oligomerisierungsgrad n im Sinne des obigen Reaktionsschemas liegt bei n = 9, die Säurezahl liegt oberhalb von 50 mg KOH/g Reaktionsprodukt. Zur Erreichung niedrigerer Säurezahlen und damit zur weitgehenden Unterdrückung von Korrosionen und außerdem zur Verschiebung der mittleren Kettenlänge des Oligoesters zu kürzeren Kettenlängen ist es zweckmäßig, in Gegenwart eines molaren Überschusses an Diol zu verestern. Verestert man nun die Dicarbonsäure unter den angegebenen Reaktionsbedingungen mit einem Diol im molaren Verhältnis von 1:2, so hat man zwar die Gewißheit, daß die freien Carboxylgruppen relativ schnell verestert sind und das erhaltene Oligoestergemisch nur noch eine mittlere Kettenlänge von beispielsweise n = 5 hat, muß aber einen relativ hohen Anteil an Diol einsetzen. Überraschenderweise wurde nun gefunden und stellt eine weitere vorteilhafte Variante des erfindungsgemäßen Verfahrens dar, daß man schon bei einem geringen molaren Überschuß an Diol, bezogen auf die Dicarbonsäure, Säurezahlen von maximal 50 mg KOH/g Reaktionsgemisch, beispielsweise Säurezahlen von 20 bis 50, erreichen kann. Ein solches molares Verhältnis für den benötigten Oligoester beträgt 1,03 bis 1,15 Mol, bevorzugt 1,05 bis 1,12 Mol Diol pro 1 Mol der Dicarbonsäure. Die hierbei erhaltenen mittleren Veresterungsgrade (Oligomerisierungsgrade) bewegen sich im Bereich von n = 3 bis n = 6.

Die eingesetzten Dicarbonsäuren und die eingesetzten Diole haben üblicherweise eine Reinheit von mehr als 99 %. Dadurch, daß auf systemfremde Stoffe verzichtet werden kann, reicht jedoch auch die Reinheit von Destillationsrückläufen aus dem der Oligoveresterung folgenden Hydrierschritt zur Durchführung des erfindungsgemäßen Verfahrens völlig aus, selbst wenn diese Destillationsrückläufe geringe Mengen nicht hydrierten Oligoesters enthalten. Solche zurückgeführten Oligoester und andere Anteile sind bei der Festlegung des genannten Molverhältnisses zwischen Diol und Dicarbonsäure zu berücksichtigen.

Es ist ein weiterer Vorteil des erfindungsgemäßen Verfahrens, daß der so hergestellte Oligoester ohne zusätzliche Reinigungsschritte direkt in der Hydrierstufe weiterverarbeitet werden kann.

Der Hydrierschritt im erfindungsgemäßen Verfahren erfolgt in der flüssigen Phase mit überschüssigem Wasserstoff, der das 20- bis 100-fache der stöchiometrisch nötigen Menge beträgt. Durch das Arbeiten in der flüssigen Phase wird der Energieaufwand für Gasphasenprozesse kostensparend verringert. Während zur Hydrierung von Estern noch vielfach ein diskontinuierliches Verfahren (Batch-Prozeß) angewendet wird, für das die bei der Veresterung geschilderten Nachteile ebenfalls gelten und bei welchem pulverförmige Katalysatoren in einem Suspensionsverfahren zum Einsatz kommen, wird das erfindungsgemäße Verfahren in seiner Hydrierstufe vollkontinuierlich betrieben. Weiterhin wird die Hydrierstufe an einem stückigen Katalysator durchgeführt. Hierdurch werden die Schwierigkeiten mit pulverförmigen Katalysatoren umgangen, nämlich die Schwierigkeit, Pulverkatalysatoren gezielt und gleichmäßig zu aktivieren, die Schwierigkeit, Pulverkatalysatoren mit Hilfe von speziellen Schlammpumpen umzulwälzen, und die Schwierigkeit, Pulverkatalysatoren vom Reaktionsprodukt quantitativ abzutrennen. Schlammpumpen unterliegen nämlich einer hohen mechanischen Beanspruchung. Die quantitative Entfernung pulverförmiger Katalysatoren ist weiterhin aufwendig, weil sie eine Grob- und eine Feinfiltration mit Apparaten in Wechselausführung erfordert. Ferner ist die Gefahr groß, daß die Katalysatoren durch diese zusätzlichen Operationen schnell ihre Aktivität verlieren und daher weiterhin hohe Katalysatorverbräuche in Kauf zu nehmen sind. Im Gegensatz zu diesen aufgezeigten Schwierigkeiten besitzen die erfindungsgemäß einzusetzenden stückigen Katalysatoren eine hohe Säureunempfindlichkeit, eine hohe Druckunempfindlichkeit und weisen eine hohe Aktivität auf, die auch über einen Zeitraum von ein bis mehreren Jahren nicht nachläßt. Der letztere Vorteil ist sehr wichtig, da auch bei stückigen, im Festbett angeordneten Katalysatoren ein häufiger Katalysatorwechsel sehr aufwendig ist.

Der erfindungsgemäß einzusetzende stückige Katalysator besteht aus verpreßten Pulvern von Cu-, Zn- und Al-Oxiden, bei denen der Cu-Anteil 40 bis 60 Gew.-%, der Zn-Anteil 15 bis 30 Gew.-% und der Al-Anteil 0,2 bis 6 Gew.-% beträgt, wobei alle Angaben auf die Gesamtmenge des Oxidpulvergemisches bezogen sind und der Rest zu 100 Gew.-% Sauerstoff darstellt. Ein solcher Katalysator kann in dieser Form ohne weitere Zusätze zum erfindungsgemäßen Hydrierschritt eingesetzt werden. In vorteilhafter Weise enthält er jedoch zusätzlich einen Gehalt von mindestens einem Oxid von Metallen der Eisengruppe des Periodensystems der Elemente (Mendelejew) und/oder des Mangans. Als Elemente der Eisengruppe kommen hierbei Eisen, Kobalt und Nickel in Frage. Oxide der Elemente Eisen, Kobalt, Nickel und Mangan, bevorzugt Eisen, Kobalt und Nickel, können sowohl einzeln als auch im Gemisch von Oxiden mehrerer der genannten Elemente eingesetzt werden. Die Gesamtmenge von Oxiden der Eisengruppe und/oder des Mangans im verpreßten Oxidpulver beträgt 0,05 bis 1,5 Gew.-%, bevorzugt 0,1 bis 0,5 Gew.-% des gesamten Oxidpulvers für die Hydrierkatalysatoren. Für den Fall des Einsatzes mehrerer Oxide von Elementen der Eisengruppe und des Mangans liegt jedes dieser gemischten Oxide in einer Menge vor, die nicht kleiner als 20 % und nicht größer als 80 % des genannten Gesamtbereiches von 0,05 bis 1,5 Gew.-% ist.

Die einzusetzenden Katalysatoren sollen möglichst frei von Alkali- und Erdalkalimetallen sein; der maximal zulässige Verunreinigungswert beträgt hier 0,1 Gew.-%, bezogen auf die Gesamtmenge des Oxidpulvergemisches.

Die Herstellung der trägerfreien stückigen Katalysatoren kann nach gebräuchlichen Methoden durch Verpressen der Metalloxidpulver, beispielsweise auf Tablettier-oder Pelletiermaschinen, unter hohem Druck erfolgen, wobei zur Verbesserung des Haftvermögens der Metalloxidpartikel auch Graphit und/oder Klebstoffe in Mengen von 0,5 bis 3 Gew.-%, bezogen auf das Gesamtgewicht der zu verpressenden Bestandteile, zum Einsatz kommen können. Beispiele für die stückige Form der Katalysatoren sind Tabletten, Kugeln oder Granulate mit Abmessungen von 2 bis 10 mm, bevorzugt 3 bis 7 mm. Tablettierte Formkörper können weiterhin zur Vergrößerung der äußeren Oberfläche mit einer axialen Durchbohrung versehen sein. Solche Formkörper haben makroskopisch betrachtet eine glatte Oberfläche. Um eine hohe Standfestigkeit (Lebensdauer) zu erreichen, müssen die stückigen Katalysatoren eine Druckfestigkeit von 50 bis 200 N, bevorzugt 75 bis 150 N, auf die Formkörperoberfläche aufweisen. Die stückigen Katalysatoren besitzen ferner eine innere Oberfläche von 10 bis 90 m²/g, bevorzugt 30 bis 80 m²/g. Die Druckfestigkeit der trägerfreien stückigen Katalysatoren kann nach DIN 50 106 bestimmt werden. Die Bestimmung der inneren Oberflächen erfolgt nach Analyt. Chem. 30 (1958), 1387-1390 bzw. nach S.J. Gregg und S.W. Sing Adsorption, Surface Area and Porosity, London 1982, Kap. 2 und 6.

Die als Hydrierkatalysatoren eingesetzten Formkörper aus verpreßten Pulvern von Cu-, Zn- und Al-Oxiden mit einem oder ohne einen Gehalt von mindestens einem Oxid von Metallen der Eisengruppe bzw. des Mangans müssen vor ihrem Einsatz sorgfältig reduziert werden. Dies geschieht durch eine Behandlung mit Wasserstoff bei 180 bis 200°C, wobei zu Beginn der Behandlung ein Gemisch aus 10 bis 15 Vol.-% H₂ und 90 bis 85 Vol.-% Inertgas (z.B. Stickstoff) eingesetzt wird und im Verlaufe der Behandlung der Inertgasanteil bis auf Null Vol.-% vermindert wird. Eine solche Behandlung wird in einem Zeitraum von ca. 15 bis 30 Stunden durchgeführt und ist beendet, wenn der Katalysator keinen Wasserstoff mehr verbraucht und infolgedessen kein Reaktionswasser mehr bildet.

Der Hydrierungsschritt im erfindungsgemäßen Verfahren wird kontinuierlich in der flüssigen Phase bei 180 bis 250°C, bevorzugt bei 190 bis 240°C und einem H₂-Druck von 100 bis 400 bar, bevorzugt 150 bis 300 bar, mit reinem Wasserstoff durchgeführt.

Während es prinzipiell gleich ist, das zu hydrierende Oligoestergemisch im Hydrierreaktor von unten nach oben oder von oben nach unten strömen zu lassen, hat es sich als vorteilhaft herausgestellt, das Oligoestergemisch von oben nach unten über den Katalysator fließen zu lassen (Rieselphase). Dabei kann das zu hydrierende Oligoestergemisch entweder gemeinsam mit dem separat eingeführten oder vorher zugemischten Wasserstoff über den Katalysator strömen (Gleichstromverfahren) oder aber dem Wasserstoff entgegengeführt werden (Gegenstromverfahren).

Der Hydrierreaktor kann entweder ein einzelnes Hochdruckrohr aus Stahl oder einer Stahllegierung sein, das mit dem stückigen Katalysator ganz oder teilweise gefüllt wird, wobei bei größeren Rohrquerschnitten auch die Anwendung der stückigen trägerfreien Katalysatoren auf Horden (Drahtkörbe oder ähnliches) nützlich sein kann; man kann jedoch auch Hochdruckrohrbündel innerhalb eines gemeinsamen Mantels anwenden, wobei die Einzelrohre wiederum mit dem trägerfreien stückigen Katalysatoren ganz oder teilweise gefüllt werden.

Die stündliche Katalysatorbelastung kann bei 200 bis 600 ml Oligoestergemisch pro Liter Katalysator liegen. Unter den genannten Reaktionsbedingungen sind hohe Katalysatorstandzeiten von 8000 bis 16 000 Stunden zu erreichen. Das den Hydrierreaktor verlassende Reaktionsgemisch besteht nach der Entspannung, bei welcher man den überschüssigen Wasserstoff abfangen und nach erfolgter Kompression und Ergänzung des verbrauchten Wasserstoffs erneut zum Einsatz bringen kann, zu mehr als 98,5 Gew.-% aus dem zu erwartenden Diol oder dem zu erwartenden Gemisch von Diolen. Es enthält an organischen Leichtsiedern maximal 0,5 Gew.-% Monoalkohole sowie an Hochsiedern maximal 0,5 Gew.-% eines höhermolekularen Rückstandes. Die Höhersieder stellen im wesentlichen nicht umgesetztes Oligoestergemisch dar und können in den Prozeß zurückgeführt werden, so daß dessen Gesamtselektivität bezüglich der Diole bei mindestens 99,0 Gew.-% liegt. Das erzeugte Diol wird nach der destillativen Entfernung von Leicht- und Hochsiedern in einer Reinheit von mindestens 99,9 Gew.-% erhalten und ist in dieser Reinheit für alle weiterverarbeitenden chemischen Prozesse einsetzbar.

### Beispiele

### Beispiel 1

In den ersten Reaktionsapparat einer Reaktionsapparatekaskade aus säurefestem Material, die aus drei hintereinandergeschalteten Reaktionsapparaten mit einem Volumen von jeweils 5 l bestand, die jeweils mit einem Schnellrührsystem üblicher Bauart (Turbinenrührer, Drehzahl: 800 bis 1200/min) und einer Destillationskolonne (10 theoret. Böden) versehen waren, wurde eine 110°C warme Lösung von 730,7 g (5 Mol) Adipinsäure in 650 g (5,5 Mol) Hexandiol-1,6 gegeben, unter Rühren schnell auf eine Reaktionstemperatur von 140 bis 150°C erhitzt und das sich bildende Reaktionswasser bei Normaldruck abdestilliert. Nach 1,5 Stunden Verweilzeit wurde das Reaktionsgemisch in den angeschlossenen zweiten Reaktionsapparat gebracht und dort bei einer Reaktionstemperatur von 150 bis 180°C unter Rühren bei Normaldruck ein weiterer Teil des Reaktionswassers abdestilliert. Nach 1,5 Stunden weiterer Verweilzeit wurde das Reaktionsgemisch schließlich in den angeschlossenen dritten Reaktionsapparat gebracht und dort bei einem Druck von 400 mbar und bei einer Reaktionstemperatur von 180 bis 190°C der Rest des Reaktionswassers entfernt. Nach weiteren 1,5 Stunden Verweilzeit (insgesamt 4,5 Stunden) war der gewünschte Veresterungsgrad erreicht. Das erhaltene oligomere Hexandiol-1,6-adipinat (1.290 g) hatte einen mittleren Grad der Oligoveresterung (= Zahl der Moleküle Adipinsäure im Oligoester) von n = 4 (gemessen durch Gelpermeabilitätschromatographie) und eine Säurezahl von 25 mg KOH/g Reaktionsgemisch. Nach der teilweisen oder gänzlichen Entleerung des ersten Reaktionsapparates konnte der Veresterungsprozeß durch Zugabe neuer Reaktionsgemischanteile quasi-kontinuierlich fortgesetzt werden.

### Beispiel 2

In den ersten Reaktionsapparat der gleichen Reaktionsapparatekaskade wie im Beispiel 1 wurde eine Lösung von 730,7 g (5 Mol) Adipinsäure in 620,5 g (5,25 Mol) Hexandiol-1,6 gegeben, unter Rühren auf eine Reaktionstemperatur von 140 bis 150°C erhitzt und das sich bildende Reaktionswasser bei Normaldruck abdestilliert. Nach 1 Stunde Verweilzeit wurde das Reaktionsgemisch in den angeschlossenen zweiten Reaktionsapparat abgelassen und dort bei einer Reaktionstemperatur von 140 bis 180°C bei Normaldruck ein weiterer Teil des Reaktionswassers abdestilliert. Nach 1 Stunde Verweilzeit wurde das Reaktionsgemisch schließlich in den dritten Reaktionsapparat abgelassen und bei einem Druck von 350 mbar und bei einer Reaktionstemperatur von 180°C der Rest des Reaktionswassers entfernt. Nach einer Reaktionszeit von weiteren 2 Stunden (insgesamt 4 Stunden) war der gewünschte Veresterungsgrad erreicht. Das erhaltene oligomere Hexandiol-1,6-adipinat (1.256 g) hatte einen mittleren Grad der Oligoveresterung von n = 5 (Molmassenverteilung gemessen durch Gelpermeabilitätschromatographie) und eine Säurezahl von 32 mg KOH/g Reaktionsgemisch. Nach der teilweisen oder gänzlichen Entleerung des ersten bzw. zweiten Reaktionsapparates konnte der Veresterungsprozeß durch Zugabe neuer Reaktionsgemischanteile quasi-kontinuierlich fortgesetzt werden.

### Beispiel 3

Ein senkrecht stehendes, wärmeisoliertes Hochdruckrohr aus nichtrostendem, säurefestem Stoff von 45 mm Innendurchmesser und 1 m Länge, das vorher mit Stickstoff sauerstofffrei gespült worden war, wurde mit 1,4 l eines durch Tablettierung von Pulvern von Kupfer-, Zink-, Aluminium- und Eisenoxiden hergestellten Hydrierungskatalysators gefüllt. Der Kupfergehalt der Tabletten lag bei 42 Gew.-%, der Zinkgehalt bei 17 Gew.-%, der Aluminiumgehalt bei 2,0 Gew.-% und der Eisengehalt bei 0,2 Gew.-%. Die Tabletten hatten bei einer Zylinderhöhe von 5 mm und einem Durchmesser von 5 mm eine Druckfestigkeit von 125 N auf die Zylindermantelfläche und eine innere Oberfläche von 68 m²/g.

Zur Aktivierung des eine Mischung von Metalloxiden enthaltenden Katalysators wurden die Tabletten zunächst 6 Stunden im Stickstoffstrom getrocknet (Temperatur: max. 200°C Menge: 5 Nm³ N₂/h). Die eigentliche Aktivierung erfolgte bei einem Stickstoffdruck von 200 bar und einer Temperatur zwischen 180 und 200°C, wobei dem Inertgas allmählich Wasserstoff zugemischt wurde, dessen Zumischungsanteil in der Anfangsphase 10 bis 15 Vol.-% nicht übersteigen durfte. Innerhalb von 24 Stunden wurde der Stickstoffanteil der Gasmischung mehr und mehr vermindert, bis schließlich reiner Wasserstoff den Reaktor durchströmte. Die Reaktion war beendet, wenn kein Reaktionswasser, das in einem nachgeschalteten Abscheider gesammelt wurde, mehr gebildet wurde.

Nach der Aktivierung des Hydrierkatalysators wurde der Wasserstoffdruck im Reaktorsystem auf 300 bar erhöht. Anschließend wurden stündlich 420 g Hexandiol-1,6-adipinat, das nach Beispiel 1 gewonnen worden war, gemeinsam mit 5 Nm³ Wasserstoff bei einem Druck von 300 bar durch das Hochdruckrohr gepumpt, wobei das Hexandiol-1,6-adipinat vor Eintritt in das Hochdruckrohr in einem vorgeschalteten elektrisch beheizten Wärmeaustauscher auf eine Temperatur von 210°C erhitzt wurde.

Das das Reaktionsrohr verlassende Reaktionsprodukt (Roh-Hexandiol-1,6) wurde in einem zweiten Wärmeaustauscher (Wasserkühler) bei 300 bar Wasserstoffdruck auf eine Temperatur <60°C abgekühlt und in einem Gasabscheider vom überschüssigen Wasserstoff, der wieder in das Hydriersystem zurückgeführt wurde, getrennt. Nach weiterer Abkühlung auf eine Temperatur <30°C und Entspannung auf Normaldruck wurde das Reaktionsprodukt gaschromatographisch untersucht. Es enthielt an organischen Leichtsiedern lediglich 0,4 Gew.-% Monoalkohole der C-Zahlen 1 bis 6 sowie an Höhersiedern 0,3 Gew.-% nicht umgesetztes Hexandiol-1,6-adipinat, so daß der Hexandiol-1,6-Gehalt des organischen Reaktionsproduktes bei 99,3 Gew.-% lag. Das erzeugte Hexandiol-1,6 wurde nach der destillativen Entfernung von Leicht- und Hochsiedern in einer Reinheit von ≥99,9 Gew.-% erhalten. Da die Höhersieder wieder in den Prozeß zurückgeführt werden konnten, lag dessen Gesamtselektivität bezüglich Hexandiol-1,6 bei 99,6 Gew.-%.

Der Katalysator war nach einer Laufzeit von 7800 Stunden unverändert wirksam, so daß sich die Zusammensetzung des Reaktionsproduktes über diesen Zeitraum nicht veränderte.

### Beispiel 4

Ein Hochdruckrohr wie in Beispiel 3 wurde unter Inertgas mit 1,4 l eines durch Tablettierung von Pulvern von Kupfer-, Zink-, Aluminium- und Nickeloxiden hergestellten Hydrierungskatalysators gefüllt. Der Kupfergehalt der Tabletten lag bei 51 Gew.-%, der Zinkgehalt bei 19 Gew.-%, der Aluminiumgehalt bei 0,5 Gew.-% und der Nickelgehalt bei 0,15 Gew.-%. Die Tabletten hatten bei einer Zylinderhöhe von 3 mm und einem Durchmesser von 3 mm eine Druckfestigkeit von 81 N auf die Zylindermantelfläche und eine innere Oberfläche von 58 m²/g.

Nach der Aktivierung des eine Mischung von Metalloxiden enthaltenden Hydrierkatalysators wie in Beispiel 3 wurde der Wasserstoffdruck auf 300 bar erhöht. Anschließend wurden stündlich 420 g Hexandiol-1,6-adipinat, das nach Beispiel 2 gewonnen worden war, gemeinsam mit 5 Nm³ Wasserstoff bei einem Druck von 300 bar kontinuierlich durch das Hochdruckrohr gepumpt, wobei das Hexandiol-1,6-adipinat vor Eintritt in das Hochdruckrohr auf eine Temperatur von 220°C erhitzt wurde.

Das das Reaktionsrohr verlassende Reaktionsprodukt (Roh-Hexandiol-1,6) enthielt nach der Abtrennung von überschüssigem Wasserstoff und der Abkühlung auf eine Temperatur <30°C laut gaschromatographischer Analyse an organischen Leichtsiedern 0,48 Gew.-% Monoalkohole der C-Zahlen 1 bis 6 sowie an Höhersiedern 0,35 Gew.-% nicht umgesetztes Hexandiol-1,6-adipinat, so daß der Hexandiol-1,6-Gehalt des organischen Reaktionsproduktes bei 99,17 Gew.-% lag. Nach der destillativen Entfernung von Leicht- und Hochsiedern wurde das erzeugte Hexandiol-1,6 in einer Reinheit von 99,9 Gew.-% erhalten. Da die Höhersieder wieder in den Prozeß zurückgeführt werden konnten, lag dessen Gesamtselektivität bezüglich Hexandiol-1,6 bei 99,52 Gew.-%.

Der Katalysator war nach einer Laufzeit von 5400 Stunden unverändert wirksam, so daß sich die Zusammensetzung des Reaktionsproduktes über diesen Zeitraum nicht veränderte.

### Beispiel 5

Ein Hochdruckrohr wie in Beispiel 3 wurde unter Inertgas mit 1,4 l eines durch Tablettierung von Pulvern von Kupfer-, Zink-, Aluminium- und Cobaltoxiden hergestellten Hydrierungskatalysators gefüllt. Der Kupfergehalt der Tabletten lag bei 49 Gew.-%, der Zinkgehalt bei 27 Gew.-%, der Aluminiumgehalt bei 1,8 Gew.-% und der Cobaltgehalt bei 0,22 Gew.-%. Die Tabletten hatten bei einer Zylinderhöhe von 5 mm und einem Durchmesser von 5 mm eine Druckfestigkeit von 110 N auf die Zylindermantelfläche und eine innere Oberfläche von 47 m²/g.

Nach der Aktivierung des oxidischen Hydrierkatalysators wie im Beispiel 3 wurde der Wasserstoffdruck auf 250 bar erhöht. Anschließend wurden stündlich 420 g Hexandiol-1,6-adipinat, das nach Beispiel 2 gewonnen worden war, gemeinsam mit 5 Nm³ Wasserstoff unter einem Druck von 250 bar kontinuierlich durch das Hochdruckrohr gepumpt, wobei das Hexandiol-1,6-adipinat vor Eintritt in das Hochdruckrohr auf eine Temperatur von 220°C erhitzt wurde. Das das Reaktionsrohr verlassende Reaktionsprodukt (Roh-Hexandiol-1,6) enthielt nach der Abtrennung von überschüssigem Wasserstoff und der Abkühlung auf eine Temperatur <30°C laut gaschromatographischer Analyse an organischen Leichtsiedern 0,68 Gew.-% Monoalkohole der C-Zahlen 1 bis 6 sowie an Höhersiedern 0,38 Gew.-% nicht umgesetztes Hexandiol-1,6-adipinat, so daß der Hexandiol-1,6-Gehalt des organischen Reaktionsproduktes bei 98,94 Gew.-% lag. Da das nicht umgesetzte Hexandiol-1,6-adipinat wieder in den Prozeß zurückgeführt werden konnte, lag dessen Gesamtselektivität bezüglich Hexandiol-1,6 bei 99,32 Gew.-%.

Der Katalysator war nach einer Laufzeit von 3800 Stunden unverändert wirksam, so daß sich die Zusammensetzung des Reaktionsproduktes über diesen Zeitraum nicht veränderte.

### Beispiel 6

In den ersten Reaktionsapparat der gleichen Reaktionsapparatekaskade wie im Beispiel 1 wurde eine Lösung von 590,5 g (5 Mol) Bernsteinsäure in 504,7 g (5,6 Mol) Butandiol-1,4 gegeben, unter Rühren auf eine Reaktionstemperatur von 130-140°C erhitzt und das sich bildende Reaktionswasser unter Normaldruck abdestilliert. Nach 2 Stunden Verweilzeit wurde das Reaktionsgemisch in den angeschlossenen zweiten Reaktionsapparat abgelassen und dort bei einer Reaktionstemperatur von 140-160°C unter Normaldruck ein weiterer Teil des Reaktionswassers abdestilliert. Nach 1 Stunde Verweilzeit wurde das Reaktionsgemisch schließlich in den dritten Reaktionsapparat abgelassen und unter einem Druck von 800 mbar und bei einer Reaktionstemperatur von 160°C der Rest des Reaktionswassers entfernt. Nach einer Reaktionszeit von weiteren 2 Stunden (insgesamt 5 Stunden) war der gewünschte Veresterungsgrad erreicht. Das erhaltene oligomere Butandiol-1,4-succinat (1.005 g) hatte einen mittleren Grad der Oligoveresterung von n = 5 (Molmassenverteilung gemessen durch Gelpermeabilitätschromatographie) und eine Säurezahl von 46 mg KOH/g Reaktionsgemisch. Nach der teilweisen oder gänzlichen Entleerung des ersten bzw. zweiten Reaktionsapparates konnte der Veresterungsprozeß durch Zugabe neuer Reaktionsgemischanteile kontinuierlich fortgesetzt werden.

### Beispiel 7

Ein senkrecht stehendes, wärmeisoliertes Hochdruckrohr aus nichtrostendem, säurefestem Stahl von 45 mm Innendurchmesser und 1 m Länge, das vorher mit Stickstoff sauerstofffrei gespült worden war, wurde mit 1,4 l eines durch Tablettierung von Pulvern von Kupfer-, Zink-, Aluminium- und Eisenoxiden hergestellten Hydrierungskatalysators gefüllt. Der Kupfergehalt der Tabletten lag bei 42 Gew.-%, der Zinkgehalt bei 17 Gew.-%, der Aluminiumgehalt bei 2,0 Gew.-% und der Eisengehalt bei 0,2 Gew.-%. Die Tabletten hatten bei einer Zylinderhöhe von 5 mm und einen Durchmesser von 5 mm eine Druckfestigkeit von 125 N auf die Zylindermantelfläche und eine innere Oberfläche von 68 m²/g.

Zur Aktivierung des eine Mischung von Metalloxiden enthaltenden Katalysators wurden die Tabletten zunächst 6 Stunden im Stickstoffstrom getrocknet (Temperatur: max. 200°C, Menge: 5 Nm³ N₂/h). Die eigentliche Aktivierung erfolgte unter einem Stickstoffdruck von 100 bar bei einer Temperatur zwischen 180 und 200°C, wobei dem Inertgas allmählich Wasserstoff zugemischt wurde, dessen Zumischungsanteil in der Anfangsphase 10-15 Vol.-% nicht übersteigen durfte. Innerhalb von 24 Stunden wurde der Stickstoffanteil der Gasmischung mehr und mehr vermindert, bis schließlich reiner Wasserstoff den Reaktor durchströmte. Die Reaktion war beendet, wenn kein Reaktionswasser, das in einem nachgeschalteten Abscheider gesammelt wurde, mehr gebildet wurde.

Nach der Aktivierung des Hydrierkatalysators wurde der Wasserstoffdruck im Reaktorsystem auf 150 bar erhöht.

Anschließend wurden stündlich 420 g Butandiol-1,4-succinat, das nach Beispiel 1 gewonnen worden war, gemeinsam mit 5 Nm³ Wasserstoff unter einem Druck von 150 bar durch das Hochdruckrohr gepumpt, wobei das Butandiol-1,4-succinat vor Eintritt in das Hochdruckrohr in einem vorgeschalteten elektrisch beheizten Wärmeaustauscher auf eine Temperatur von 210°C erhitzt wurde.

Das das Reaktionsrohr verlassende Reaktionsprodukt (Roh-Butandiol-1,4) wurde in einem zweiten Wärmeaustauscher (Wasserkühler) unter 300 bar Wasserstoffdruck auf eine Temperatur < 60°C abgekühlt und in einem Gasabscheider vom überschüssigen Wasserstoff, der wieder in das Hydriersystem zurückgeführt wurde, getrennt.

Nach weiterer Abkühlung auf eine Temperatur <30°C und Entspannung auf Normaldruck wurde das Reaktionsprodukt gaschromatographisch untersucht.

Es enthielt an organischen Leichtsiedern 0,2 Gew.-% Tetrahydrofuran und 0,1 Gew.-% n-Butanol sowie an Höhersiedern 0,4 Gew.-% nicht umgesetztes Butandiol-1,4-succinat, so daß der Butandiol-1,4-Gehalt des organischen Reaktionsproduktes bei 99,3 Gew.-% lag.

Das erzeugte Butandiol-1,4 wurde nach der destillativen Entfernung von Leicht-und Hochsiedern in einer Reinheit von ≥ 99,9 Gew.-% erhalten.

Da die Höhersieder wieder in den Prozeß zurückgeführt werden konnten, lag dessen Gesamtselektivität bezüglich Butandiol-1,4 bei 99,7 Gew.-%.

Der Katalysator war nach einer Laufzeit von 5.600 Stunden unverändert wirksam, so daß sich die Zusammensetzung des Reaktionsproduktes über diesen Zeitraum nicht veränderte.

## Patentansprüche

1. Verfahren zur Herstellung von aliphatischen α,ω-Diolen mit 4 bis 12 C-Atomen aus aliphatischen α,ω-Dicarbonsäuren mit 4 bis 12 C-Atomen durch Oligoveresterung der Dicarbonsäuren mit den Diolen und katalytische Hydrierung des entstandenen Oligoesters in der Flüssigphase, dadurch gekennzeichnet, daß der Oligoester kontinuierlich bei 180 bis 250°C und einem H₂-Druck von 100 bis 400 bar wobei die H₂-Menge das 20- bis 100-fache der stöchiometrisch benötigten Menge beträgt, an einem stückigen reduzierten Katalysator, vor der Reduktion bestehend aus verpreßten Pulvern von Cu-, Zn- und Al-Oxiden mit einem oder ohne einen Gehalt von mindestens einem Oxid von Metallen der Eisengruppe des Periodensystems der Elemente (Mendelejew) oder des Mangans, hydriert wird.

2. Verfahren nach Anspruch 1, dadurch gekennzeichnet, daß die Oligoveresterung diskontinuierlich oder quasi-kontinuierlich in 1 bis 4 Veresterungsstufen, bevorzugt quasi-kontinuierlich in 2 oder 3 Veresterungsstufen unter destillativer Entfernung des Reaktionswassers im Temperaturbereich von 100 bis 240°C und im Druckbereich von 1500 bis 100 mbar durchgeführt wird.

3. Verfahren nach Anspruch 2, dadurch gekennzeichnet, daß bei mehrstufiger Oligoveresterung die Temperatur von Stufe zu Stufe um 10 bis 40°C, bevorzugt um 15 bis 30°C, und innerhalb jeder Stufe um 5 bis 30°C gesteigert wird.

4. Verfahren nach Anspruch 2, dadurch gekennzeichnet, daß bei mehrstufiger Oligoveresterung die erste Stufe bei Normaldruck, die letzte Stufe bei 200 bis 600 mbar und im Falle von 3 oder 4 Stufen die mittlere(n) Stufe(n) bei 400 bis 1000 mbar durchgeführt wird (werden).

5. Verfahren nach Anspruch 1, dadurch gekennzeichnet, daß der eingesetzte Oligoester aus einem Gemisch von 1,03 bis 1,15 Mol, bevorzugt 1,05 bis 1,12 Mol Diol pro 1 Mol Dicarbonsäure so hergestellt wird, daß ein durchschnittlicher Veresterungsgrad von 3 bis 6 und eine Säurezahl von 20 bis 50 mg KOH/g Oligoester erreicht wird.

6. Verfahren nach Anspruch 1, dadurch gekennzeichnet, daß der eingesetzte Oligoester ohne weitere Reinigung zur Hydrierung eingesetzt wird.

7. Verfahren nach Anspruch 1, dadurch gekennzeichnet, daß bei dem stückigen Katalysator vor der Reduktion im Gemisch der verpreßten Oxidpulver der Cu-Anteil 40 bis 60 Gew.-%, der Zn-Anteil 15 bis 30 Gew.-% und der Al-Anteil 0,2 bis 6 Gew.-% beträgt, wobei alle Angaben auf die Gesamtmenge des Oxidpulvergemisches bezogen sind und der Rest zu 100 Gew.-% Sauerstoff darstellt.

8. Verfahren nach Anspruch 1, dadurch gekennzeichnet, daß in dem stückigen Katalysator vor der Reduktion eines oder eines oder mehrere Oxide der Eisengruppe oder des Mangans im verpreßten Oxidpulver vorliegen und deren Gesamtmenge 0,05 bis 1,5 Gew.-%, bevorzugt 0,1 bis 0,5 Gew.-% des gesamten Oxidpulvers beträgt.

9. Verfahren nach Anspruch 1, dadurch gekennzeichnet, daß der stückige, reduzierte Katalysator Zylinder- oder Kugelform mit Abmessungen von 2 bis 10 mm, bevorzugt 3 bis 7 mm hat und eine Druckfestigkeit von 50 bis 200 N, bevorzugt 75 bis 150 N, auf die Formkörperoberfläche und eine innere Oberfläche von 10 bis 90 m² /g, bevorzugt 30 bis 80 m²/g aufweist.

10. Verfahren nach Anspruch 1, dadurch gekennzeichnet, daß der Katalysator aus verpreßtem Oxidpulver vor der Hydrierung durch eine Behandlung mit H₂ bei 180 bis 200°C aktiviert wird, wobei zu Beginn der Behandlung ein Gemisch aus 10 bis 15 Vol.-% H₂ und 90 bis 85 Vol.-% Inertgas eingesetzt wird und im Verlaufe der Behandlung der Inertgasanteil bis auf Null Vol.-% vermindert wird.

## Claims

1. Process for the preparation of aliphatic α,ω-diols of 4 to 12 carbon atoms from aliphatic α,ω-dicarboxylic acids of 4 to 12 carbon atoms by oligoesterification of the said dicarboxylic acids with the said diols and catalytic hydrogenation of the resulting oligo ester in the liquid phase, characterized in that the oligo ester is hydrogenated continuously at from 180 to 250°C and at an H₂ pressure of from 100 to 400 bar, the quantity of H₂ being from 20 to 100 times the stoichiometrically required quantity, over a reduced catalyst in piece form consisting, prior to the reduction, of compressed powders of Cu oxides, Zn oxides and Al oxides which do or do not contain at least one oxide of metals of the iron group of the Periodic Table of the Elements (Mendeleev) or of manganese.

2. Process according to Claim 1, characterized in that the oligoesterification is carried out batchwise or quasi-continuously in 1 to 4 esterification stages, preferably quasi-continuously in 2 or 3 esterification stages, with distillative removal of the water of reaction in the temperature range from 100 to 240°C and in the pressure range from 1500 to 100 mbar.

3. Process according to Claim 2, characterized in that, in the case of multistage oligoesterification, the temperature is increased from stage to stage by from 10 to 40°C, preferably by from 15 to 30°C, and within each stage by from 5 to 30°C.

4. Process according to Claim 2, characterized in that, in the case of multistage oligoesterification, the first stage is carried out at atmospheric pressure, the last stage at from 200 to 600 mbar and, in the case of 3 or 4 stages, the middle stage(s) at from 400 to 1000 mbar.

5. Process according to Claim 1, characterized in that the oligo ester employed is prepared from a mixture of from 1.03 to 1.15 mol, preferably from 1.05 to 1.12 mol, of diol per mole of dicarboxylic acid in such a way that an average degree of esterification of from 3 to 6 and an acid number of from 20 to 50 mg of KOH/g of oligo ester are achieved.

6. Process according to Claim 1, characterized in that the oligo ester employed is used without further purification for hydrogenation.

7. Process according to Claim 1, characterized in that, in the catalyst in piece form prior to the reduction, in the mixture of the compressed oxide powders, the Cu proportion is from 40 to 60% by weight, the Zn proportion is from 15 to 30% by weight and the Al proportion is from 0.2 to 6% by weight, all figures being based on the total quantity of the oxide powder mixture, and the remainder to 100% by weight constituting oxygen.

8. Process according to Claim 1, characterized in that, in the catalyst in piece form prior to the reduction, one or more oxides of the iron group or of manganese are present in the compressed oxide powder, and the total quantity thereof is from 0.05 to 1.5% by weight, preferably from 0.1 to 0.5% by weight of the total oxide powder.

9. Process according to Claim 1, characterized in that the reduced catalyst in piece form has a cylindrical or spherical form with dimensions of from 2 to 10 mm, preferably from 3 to 7 mm and a compressive strength of from 50 to 200 N, preferably from 75 to 150 N, on the shaped-body surface and an internal surface area of from 10 to 90 m²/g, preferably from 30 to 80 m²/g.

10. Process according to Claim 1, characterized in that the catalyst of compressed oxide powder is activated prior to hydrogenation by treatment with H₂ from 180 to 200°C, employing at the beginning of the treatment a mixture of from 10 to 15% by volume of H₂ and from 90 to 85% by volume of inert gas and, in the course of the treatment, reducing the inert gas component down to zero % by volume.

## Revendications

1. Procédé de préparation d'α,ω-diols aliphatiques ayant 4 à 12 atomes C à partir d'acides α,ω-dicarboxyliques aliphatiques, ayant 4 à 12 atomes C, par oligoestérification du diacide carboxylique avec le diol et hydrogénation catalytique de l'oligoester formé en phase liquide, caractérisé en ce que l'oligoester est hydrogéné de manière continue, entre 180 et 250°C, et à une pression en H₂ allant de 100 à 400 bars, et la quantité de H₂ se situe dans l'intervalle allant de 20 à 100 fois la quantité stoechiométrique nécessaire, sur un catalyseur réduit en morceaux, consistant avant la réduction en des poudres comprimées d'oxydes de Cu, Zn et Al avec une teneur ou sans teneur en au moins un oxyde des métaux du groupe du fer du système périodique des éléments (Mendeleïev) ou de manganèse.

2. Procédé suivant la revendication 1, caractérisé en ce que l'oligoestérification est réalisée de manière discontinue ou quasi continue en 1 à 4 étapes d'estérification, de préférence de manière quasi continue en 2 ou 3 étapes d'estérification, avec séparation par distillation de l'eau de réaction, dans un intervalle de température allant de 100 à 240°C et dans un intervalle de pression allant de 1500 à 100 mbars.

3. Procédé suivant la revendication 2, caractérisé en ce que lors de l'oligoestérification en plusieurs étapes, la température est augmentée étape par étape de 10 à 40°C, de préférence de 15 à 30°C, et au sein de chaque étape, chaque fois de 5 à 30°C.

4. Procédé suivant la revendication 2, caractérisé en ce que lors de l'oligoestérification à plusieurs étapes, la première étape est réalisée à pression normale, la dernière étape entre 200 et 600 mbars et dans le cas de 3 ou 4 étapes, la ou les étapes intermédiaires entre 400 et 1000 mbars.

5. Procédé suivant la revendication 1, caractérisé en ce que l'oligoester mis en oeuvre est préparé à partir d'un mélange de 1,03 à 1,15 mole, de préférence de 1,05 à 1,12 mole de diol par mole de diacide carboxylique, de sorte qu'un taux moyen d'estérification de 3 à 6 et un indice acide de 20 à 50 mg de KOH/g d'oligoester soit atteint.

6. Procédé suivant la revendication 1, caractérisé en ce que l'oligoester introduit est mis en oeuvre dans l'hydrogénation sans autre purification.

7. Procédé suivant la revendication 1, caractérisé en ce que, pour les catalyseurs en morceaux avant la réduction, dans le mélange de poudres d'oxydes comprimées, la quantité de Cu se situe dans l'intervalle allant de 40 à 60% en poids, la quantité de Zn de 15 à 30% en poids et la quantité de Al de 0,2 à 6% en poids, où toutes les données se rapportent à la quantité totale du mélange pulvérulent d'oxydes et le reste représente de l'oxygène pour faire 100% en poids.

8. Procédé suivant la revendication 1, caractérisé en ce que, dans le catalyseur en morceaux avant la réduction, un ou plusieurs oxydes du groupe du fer ou de manganèse est présent dans la poudre d'oxydes comprimée et dont la quantité totale se situe dans l'intervalle allant de 0,05 à 1,5% en poids, de préférence, de 0,1 à 0,5% en poids de la poudre d'oxyde totale.

9. Procédé suivant la revendication 1, caractérisé en ce que le catalyseur en morceaux, réduit, a la forme de cylindres ou de sphères avec des dimensions de 2 à 10 mm, de préférence 3 à 7 mm et une résistance à la pression de 50 à 200 M, de préférence 75 à 150 N, sur la surface de l'article moulé et une surface interne de 10 à 90 m²/g, de préférence 30 à 80 m²/g.

10. Procédé suivant la revendication 1, caractérisé en ce que le catalyseur en poudre d'oxydes comprimée, est activé avant l'hydrogénation par un traitement avec du H₂ entre 180 et 200°C, où pour commencer le traitement, on met en oeuvre un mélange constitué de 10 à 15% en volume de H₂ et de 90 à 85% en volume d'un gaz inerte et au cours du traitement, la quantité de gaz inerte est diminuée jusqu'à 0% en volume.
